(19) Europäisches Patentamt
European Patent Office
Office européen des brevets

(11) **EP 2 477 568 B1**

(12) **EUROPÄISCHE PATENTSCHRIFT**

(45) Veröffentlichungstag und Bekanntmachung des Hinweises auf die Patenterteilung:
**30.03.2016 Patentblatt 2016/13**

(21) Anmeldenummer: **10766236.3**

(22) Anmeldetag: **18.08.2010**

(51) Int Cl.:
*A61B 18/20* (2006.01)    *A61C 1/00* (2006.01)

(86) Internationale Anmeldenummer:
**PCT/DE2010/075079**

(87) Internationale Veröffentlichungsnummer:
**WO 2011/032551 (24.03.2011 Gazette 2011/12)**

(54) **LASERSTRAHL-AUSRICHTEINHEIT UND LASERBEARBEITUNGSGERÄT ZUR BEARBEITUNG EINES MATERIALS**

LASER BEAM ALIGNING UNIT AND LASER TREATMENT DEVICE FOR TREATING A MATERIAL

UNITÉ D'ORIENTATION DE FAISCEAU LASER ET APPAREIL DE TRAITEMENT LASER DESTINÉ AU TRAITEMENT D'UNE MATIÈRE

(84) Benannte Vertragsstaaten:
**AL AT BE BG CH CY CZ DE DK EE ES FI FR GB GR HR HU IE IS IT LI LT LU LV MC MK MT NL NO PL PT RO SE SI SK SM TR**

(30) Priorität: **18.09.2009 DE 102009042199**

(43) Veröffentlichungstag der Anmeldung:
**25.07.2012 Patentblatt 2012/30**

(73) Patentinhaber: **Lumera Laser GmbH**
**67661 Kaiserslautern (DE)**

(72) Erfinder: **KASENBACHER, Anton**
**83278 Traunstein (DE)**

(74) Vertreter: **Patentanwälte Lambsdorff & Lange**
**Dingolfinger Strasse 6**
**81673 München (DE)**

(56) Entgegenhaltungen:
**WO-A1-2005/009267**    **WO-A2-2006/074469**
**US-A1- 2004 176 718**    **US-E1- R E37 585**
**US-E1- R E37 585**

**Beschreibung**

Die vorliegende Erfindung betrifft eine Laserstrahl-Ausrichteinheit zur Bearbeitung eines Materials, ein Laserbearbeitungsgerät zur Bearbeitung eines Materials und ein Verfahren zur Bearbeitung von Gewebe.

Die Eigenschaften der Laserstrahlung, insbesondere die Intensität und gute Fokussierbarkeit, haben dazu geführt, dass Laser heutzutage in vielen Gebieten der Materialbearbeitung zum Einsatz kommen. Bei Laser-Substrat-Wechselwirkungen wird der Photonenstrahl von dem zu bearbeitenden Werkstück absorbiert, wobei in Abhängigkeit von der Energie das Material angeregt und/oder erhitzt und/oder verdampft und/oder dissoziiert und/oder ionisiert wird. Bei Laserbearbeitungsverfahren wird der Laserstrahl von dem zu bearbeitenden Werkstück absorbiert und durch die hohe Energie des Laserstrahls wird Material des Werkstücks erhitzt und aufgeschmolzen oder verdampft. Auf diese Weise können beispielsweise Löcher in Metallplatten gebohrt oder Halbleiter-Wafer in einzelne Halbleiterchips separiert werden. Die Laserbearbeitung erfolgt üblicherweise mittels gepulster Laserstrahlung in speziellen Laserbearbeitungsvorrichtungen. Als Laserstrahlquellen werden üblicherweise $CO_2$- oder Festkörperlaser, wie beispielsweise Nd:YAG-, Nd:YVO$_4$- oder Nd:GdVO$_4$-Laser verwendet. Ein wichtiges Merkmal eines Laserbearbeitungsverfahrens ist der Durchsatz, d.h. beispielsweise die Anzahl an Löchern, die innerhalb einer Zeiteinheit in eine Metallplatte gebohrt werden können. Dies hängt wesentlich von einer ungestörten Wechselwirkung der Laserphotonen mit dem Substrat ab. Neben anderen Aspekten besteht hier ein ständiges Bestreben nach Erhöhung der Effizienz bei gleichzeitig verringertem Energieeinsatz, d.h. Reduktion kostenintensiver Photonen.

US 2004/176718 A1 offenbart eine Laserstrahl-Ausrichteinheit zur Bearbeitung eines Materials, umfassend die Merkmale der Präambel der Ansprüche 1 und 2 der vorliegenden Anmeldung. US R E37 585 E1 erwähnt Schäden an einem transparenten Material im Rayleigh Bereich durch eine Laserstrahl-Ausrichteinheit zur Bearbeitung eines Materials.

Wenn in dieser Anmeldung von Materialbearbeitung die Rede ist, so sollen damit prinzipiell Materialien aller Art umfasst sein, d.h. insbesondere Materialien aller Aggregatzustände, Materialien natürlicher oder synthetischer Art sowie anorganische oder organische Materialien. Ein Teilbereich dieser Materialien sind biologische Gewebe wie devitale und vitale biologische Gewebe, die auch Hartgewebe wie etwa Zahnmaterial umfassen. Ein beispielhaftes Anwendungsgebiet der vorliegenden Anmeldung betrifft somit das Gebiet der Zahnheilkunde, wobei anstelle eines mechanischen Bohrers zur Ablation bzw. Abtragung von Zahnmaterial, insbesondere kariösem Zahnmaterial, ein Laserbearbeitungs-Verfahren und ein entsprechendes Laserbearbeitungsgerät zum Einsatz kommen können. Es können jedoch ebenso andere Arten von biologischen Geweben und Gewebetypen, wie beispielsweise andere Arten von Hartgewebe, Weichgewebe und Gewebeflüssigkeiten, betroffen sein. Ein anderes potentielles Anwendungsgebiet kann dementsprechend beispielsweise das Gebiet der Augenheilkunde sein.

Ein weiterer bedeutender Aspekt dieser Anmeldung besteht in der Gewährleistung maximaler sogenannter Biosicherheit (Biosafety) bei Durchführung der hier beschriebenen Bearbeitungsverfahren, insbesondere in der Form der Vermeidung der Auswirkungen exzessiver ultravioletter Strahlung. Es ist bekannt, dass bei einer Laserbearbeitung der in dieser Anmeldung beschriebenen Art jeder Laserpuls mit einem dünnen Oberflächenbereich des Materials derart wechselwirken soll, dass im Fokus des Bearbeitungs-Laserstrahls ein Mikroplasma gebildet wird. Dieses Plasma kann ggf. Ursache von thermischen (z.B. Dehydratation) und/oder chemischen (z.B. ultraviolette Strahlung) Schädigungen sein. Derartige Stressfaktoren können, wie allgemein bekannt ist, gesundheitsschädigende Auswirkungen haben. Dies gilt natürlich in besonderem Masse für den hier bereits genannten Anwendungsfall der Zahnbehandlung. Es besteht somit ein generelles Ziel darin, diese exogenen und/oder endogenen Noxen unter Beibehaltung größtmöglicher Ablationseffizienz zu vermeiden.

Es ist demgemäß Aufgabe der vorliegenden Erfindung, eine Laserstrahl-Ausrichteinheit und ein Laserbearbeitungsgerät anzugeben, mit welchen eine effiziente Bearbeitung eines Materials gewährleistet werden kann, insbesondere eine effiziente Ausnutzung der von der Laserstrahlquelle gelieferten Laserstrahlung, d.h. Photonenschutz und Photonenreduktion. Eine weitere Aufgabe der vorliegenden Erfindung ist die Angabe eines Verfahrens zur Bearbeitung eines Gewebes, das die bei der Bearbeitung ggf. auftretenden Nebenwirkungen umgeht, zumindest aber beherrschbar macht.

Diese Aufgaben werden mit den Merkmalen der unabhängigen Patentansprüche gelöst. Vorteilhafte Weiterbildungen und Ausgestaltungen sind Gegenstand von nebengeordneten Ansprüchen oder Unteransprüchen.

Eine erste Erkenntnis der vorliegenden Erfindung besteht darin, dass bei der Bearbeitung bzw. Ablation von Materialien mit einem Laserstrahl das ablatierte Material, auch wenn es geeignet abgesaugt wird, teilweise eine optische Barriere für den Laserstrahl bilden kann, da ablatierte Partikel sich für unbestimmte Zeit im Strahlengang des Laserstrahls befinden können und somit als Absorptions- und Streuzentren für den Laserstrahl wirken können. Es ist somit eine Anordnung anzustreben, die es erlaubt, den Laserstrahl weitgehend ungehindert bis zu der Oberfläche des zu bearbeitenden Materials gelangen zu lassen.

Eine weitere Erkenntnis der vorliegenden Erfindung besteht darin, dass bei der Materialbearbeitung ggf. thermische und/oder chemische Schädigungen entstehen können. Insbesondere bei der Zahnbehandlung erfordern diese Auswirkungen adäquate Schutzmaßnahmen.

Gemäß einem ersten Aspekt der vorliegenden Erfindung wird eine Laserstrahl-Ausrichteinheit zur Bearbeitung eines Materials angegeben, welche eine erste, innere Hülse und eine zweite, äussere Hülse aufweist, wobei die innere Hülse innerhalb der äußeren Hülse derart angeordnet ist, dass der Laserstrahl durch ihren inneren Hohlraum in Richtung auf den zu bearbeitenden Bereich des Materials führbar ist.

Eine solche Laserstrahl-Ausrichteinheit kann als ein Handstück oder ein Teil davon ausgebildet sein, welches wiederum Bestandteil eines Laserbearbeitungsgeräts sein kann, welches im Übrigen eine Laserstrahlquelle zum Bereitstellen eines gepulsten Bearbeitungs-Laserstrahls aufweist.

Eine derartige Anordnung macht es möglich, den Laserstrahl nahezu ungehindert auf die Oberfläche des zu bearbeitenden Materials zu fokussieren. Die erfindungsgemäße Anordnung ermöglicht es insbesondere, die Laserphotonen bis in den Fokusbereich hinein vor frühzeitigen Interaktionen mit z.B. Ablationsprodukten und/oder Luftpartikeln zu schützen. In letzter Konsequenz ermöglicht es die erfindungsgemäße Anordnung somit, die Laserstrahlquelle mit geringerer optischer Ausgangsleistung zu betreiben und somit Energie einzusparen.

Gemäß einer Ausführungsform der Laserstrahl-Ausrichteinheit überragt die äussere Hülse das lichtaustrittsseitige Ende der inneren Hülse um eine vorgegebene Wegstrecke in Achsenrichtung der Hülsen. Dabei kann die vorgegebene Wegstrecke der Rayleigh-Länge in Bezug auf den Fokus des aus dem lichtaustrittsseitigen Ende der inneren Hülse austretenden Laserstrahlbündels entsprechen. Zusätzlich aber auch unabhängig davon kann die äußere Hülse so ausgebildet sein, dass sie bei der Bearbeitung mit ihrem Rand auf die Oberfläche des zu bearbeitenden Materials aufgestützt werden kann, sodass der lichtaustrittsseitige Rand der inneren Hülse um die Rayleigh-Länge von der Oberfläche des zu bearbeitenden Materials beabstandet ist. Wie weiter unten noch gezeigt werden wird, können mit einer derartigen Anordnung optimale Fokussierungsbedingungen geschaffen werden. Gleichzeitig kann dafür Sorge getragen werden, dass ein auf der lichtaustrittsseitigen Stirnseite der äusseren Hülse aufgebrachtes Schutzglas sich nicht mehr innerhalb der Fokuslänge des fokussierten Laserstrahls befindet und somit die Gefahr minimiert wird, dass das Schutzglas infolge seiner unvermeidlich vorhandenen Restabsorption durch den Laserstrahl zerstört werden könnte oder durch Multiphotonenabsorption ablatiert wird. Der Abstand zwischen dem lichtaustrittsseitigen Ende der inneren Hülse und dem Rand der äusseren Hülse kann somit gegebenenfalls auch größer als die Rayleigh-Länge gewählt werden, um die Gefahr der Zerstörung des Schutzglases durch den Laser weiter zu minimieren. Ein weiteres Kriterium für den zu bestimmenden Abstand zwischen den lichtaustrittsseitigen Enden der Hülsen und somit dem Abstand zwischen dem Schutzglas und der zu bearbeitenden Oberfläche ist die Gefahr der Verschmutzung des Schutzglases durch ablatiertes Material. Auch diesbezüglich kann es angebracht sein, einen größeren Abstand zu wählen als es aus Gründen der optischen Zerstörung geboten wäre. Allerdings kann diesbezüglich auch ein Reinigungsmechanismus vorgesehen sein, worauf weiter unten noch eingegangen wird.

Gemäß einer Ausführungsform der Laserstrahl-Ausrichteinheit sind die erste Hülse und/oder die zweite Hülse zylinder- oder konusförmig ausgebildet und weisen insbesondere einen sich in Richtung auf das lichtaustrittsseitige Ende stetig verringernden Durchmesser auf.

Gemäß einer Ausführungsform des Laserbearbeitungsgeräts weist dieses ferner eine Scan-Einheit zum Abrastern oder Scannen eines Bereichs mit dem Laserstrahl auf, wobei die Scan-Einheit, die Führungsoptik für den Laserstrahl und die Abmessungen der inneren Hülse derart aufeinander abgestimmt sind, dass der gescannte Bereich beim Durchtritt des Laserstrahls durch die innere Hülse stets innerhalb der inneren Hülse zu liegen kommt. Insofern ist also der Durchmesser und/oder Querschnitt der inneren Hülse darauf beschränkt, dass er nicht kleiner werden darf als ein gewünschter Scan-Bereich des Laserstrahls. Umgekehrt kann gesagt werden, dass die Scan-Einheit stets so anzusteuern ist, dass der von ihr erzeugte Scan-Bereich des Laserstrahls innerhalb der inneren Hülse liegt.

Gemäß einer Ausführungsform der Laserstrahl-Ausrichteinheit ist in eine Wand der äusseren Hülse und/oder in eine Wand der inneren Hülse mindestens eine Zuführungs- oder Absaugleitung integriert. Die Zuführungs- oder Absaugleitung kann dann beispielsweise in Längsrichtung der Hülse bis zu ihrem jeweiligen lichtaustrittsseitigen Ende verlaufen, sodass das Ende der Leitung dem zu bearbeitenden Oberflächenbereich direkt gegenüberliegt. Gegebenenfalls kann an diesem Ende die Leitung mit einer Düse verbunden sein, die gegebenenfalls steuer- und/oder ausrichtbar ist. Es kann beispielsweise vorgesehen sein, dass in die innere Hülse ein oder mehrere Zuführungsleitungen integriert sind und in die äußere Hülse eine Absaugleitung integriert ist. Die Zuführungsleitungen können für die Zuführung verschiedener Medien ausgelegt sein, wobei insbesondere konventionelle Medien wie Luft oder Wasser, jedoch auch spezielle Medien wie pharmazeutische Substanzen, alle Arten von organischen oder anorganischen Stoffen, freie Elektronen oder kalte Plasmen verwendet werden können.

Eine weitere Erkenntnis der vorliegenden Erfindung besteht darin, dass bei der Bearbeitung von Gewebe mit gepulster Laserstrahlung schädlicher thermischer und/oder chemischer Stress entstehen kann und insbesondere bei einer Zahnbehandlung als Kautel ein geeignetes pharmazeutisches Medium in einem Umgebungsbereich der zu bearbeitenden Zahnoberfläche applizierbar ist. Es wurde herausgefunden, dass eine Substanz namens Ectoin und Derivate davon einen geeigneten Schutz vor der bei der Laserbearbeitung evtl. entstehenden Noxen bilden.

Gemäß einem zweiten Aspekt der vorliegenden Erfindung wird somit ein Laserbearbeitungsgerät zur Bearbeitung von Gewebe angegeben, welches eine Laserstrahlquelle zum Bereitstellen eines gepulsten Bearbeitungs-Laserstrahls und eine Ausgabeeinrichtung für die Ausgabe einer Ectoin-Lösung aufweist.

Des Weiteren wird ein Verfahren zur Bearbeitung von Gewebe angegeben, bei welchem ein gepulster Laserstrahl bereitgestellt wird, eine Ectoin-Lösung bereitgestellt wird, das Gewebe in einer räumlichen Umgebung eines zu bearbeitenden Bereichs mit der Ectoin-Lösung behandelt wird und der zu bearbeitende Bereich mit dem gepulsten Laserstrahl bestrahlt wird.

Gemäß einer Ausführungsform des Verfahrens wird die Ectoin-Lösung als eine erstmals isotone Ectoin-Lösung bereitgestellt, d.h. mit gleichem osmotischen Druck wie das menschliche Blut. Es gelang, den zur Rezeptur unabdingbaren Osmolalitätswert zu ermitteln mit 78 mosm/kg. Auf Basis dieser Erkenntnis sind nun isotone Rezepturen generierbar in einem Konzentrationsbereich von $0 = n = 3,588087$ % Ectoin. Dies bedeutet, dass einer 1%-igen Ectoin-Lösung 0,646682 % NaCl zuzusetzen sind.

Gemäß einer Ausführungsform des Verfahrens wird die Ectoin-Lösung in Form eines Aerosols bereitgestellt. Dabei kann die Größe der Aerosol-Partikel in einem Bereich zwischen 0,01 $\mu$m und 2 $\mu$m liegen.

Gemäß einer Ausführungsform des Verfahrens kann eine Vorrichtung gemäß dem ersten Aspekt der Erfindung zur Durchführung des Verfahrens verwendet werden. Insbesondere kann auch als eine weitere Ausführungsform des Laserbearbeitungsgeräts dieses mit einer Ausgabeeinrichtung für die Ectoin-Lösung verbunden sein. Ferner kann insbesondere eine der bereits weiter oben beschriebenen Durchführungs-Leitungen dafür vorgesehen sein, vor oder während der Behandlung eine Ectoin-Lösung zuzuführen und mittels einer steuer- und ausrichtbaren Düse in gezielter Weise auf einen räumlichen Bereich des Gewebes in der Umgebung des zu bearbeitenden Bereichs zu applizieren.

Im Folgenden wird die Erfindung in Form weiterer Ausführungsformen anhand der Zeichnungen näher erläutert. Es zeigen:

Fig. 1          eine schematische Darstellung einer Laserstrahl-Ausrichteinheit in einem Längsschnitt gemäß einer Ausführungsform;

Fig. 2A,B     die Ausrichteinheit der Fig. 1 in einem Querschnitt entlang der Linie A-A (A) und in einem Querschnitt entlang der Linie B-B (B); und

Fig. 3          eine schematische Darstellung eines Laserbearbeitungsgeräts gemäß einer Ausführungsform.

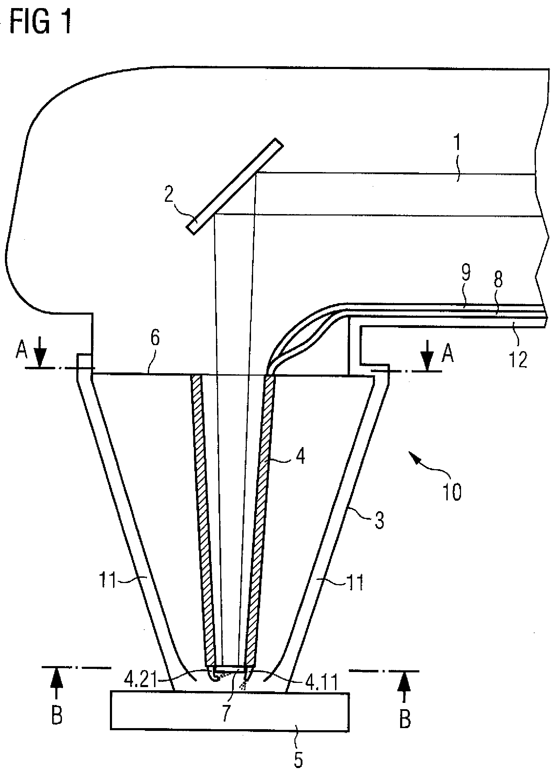

In der Fig. 1 ist eine Laserstrahl-Ausrichteinheit in einem Längsschnitt gemäß einer Ausführungsform dargestellt. Die Laserstrahl-Ausrichteinheit 10 dient dazu, einen Bearbeitungs-Laserstrahl 1 auf ein Werkstück 5 zu richten. Die Laserstrahl-Ausrichteinheit 10 kann dabei - wie dargestellt - einen horizontalen Abschnitt aufweisen, der parallel zur Oberfläche des Werkstücks 5 ausgerichtet ist und von dem lediglich ein Teil im oberen Bereich der Fig. 1 gezeigt ist. An den horizontalen Abschnitt schließt sich ein vertikaler Abschnitt an, welcher vollständig dargestellt ist und vertikal in Bezug auf die Oberfläche des Werkstücks 5 ausgerichtet ist. Der von einer Laserstrahlquelle (nicht dargestellt) kommende Bearbeitungs-Laserstrahl 1 wird durch den horizontalen Abschnitt geführt und trifft dort auf eine Umlenkeinheit 2, mittels der er in den vertikalen Abschnitt geführt wird und unterhalb des vertikalen Abschnitts auf die Oberfläche des Werkstücks 5 auftrifft. Der horizontale Abschnitt kann nicht gezeigte Mittel zum Fokussieren des Laserstrahls 1, insbesondere ein Objektiv oder eine Linse aufweisen, durch die der Laserstrahl derart fokussiert werden kann, dass sein Fokus auf der Oberfläche des Werkstücks 5 zu liegen kommt. Die Laserstrahl-Ausrichteinheit 10 kann ferner in ihrem horizontalen Abschnitt Mittel zum Scannen oder Abrastern des Laserstrahls aufweisen, durch die der Laserstrahl 1 über einen vorgegebenen, zu bearbeitenden Bereich der Oberfläche des Werkstücks 5 gescannt werden kann. Die Darstellung der Fig. 1 zeigt den Scan-Bereich des Laserstrahls 1 in einer Seitenansicht und auf der Oberfläche des Werkstücks 5 kann der Scan-Bereich beispielsweise eine rechteckige oder quadratische Form aufweisen.

Der vertikale Abschnitt der Laserstrahl-Ausrichteinheit 10 weist eine äussere Hülse 3 und eine innere Hülse 4 auf. Die innere Hülse 4 ist innerhalb der äußeren Hülse 3 derart angeordnet, dass der Laserstrahl 1 durch ihren inneren Hohlraum in Richtung auf einen zu bearbeitenden Bereich des Werkstücks 5 führbar ist.

Die äußere Hülse 3 und die innere Hülse 4 können im Prinzip jede beliebige geometrische Form aufweisen. In einer bevorzugten Ausführungsform sind sie jedoch, wie dargestellt, zylindersymmetrisch um die Strahlachse des Laserstrahls 1 ausgebildet und weisen beide einen sich kontinuierlich verringernden Querschnitt in Strahlrichtung auf. Die innere Hülse 4 ist an einer Blende 6 mittig angebracht, die mit der Innenwand der äußeren Hülse 3 oder eine darüber liegende Innenwand des oberen Abschnitts der Ausrichteinheit 10 verbunden ist. Die innere Hülse 4 kann insbesondere mit der Blende 6 einstückig ausgebildet sein und beispielsweise aus Metall gefertigt sein. Das lichtaustrittsseitige Ende der äußeren Hülse 3 überragt dasjenige der inneren Hülse 4 um eine vorgegebene Wegstrecke. Dabei kann das lichtaustrittsseitige Ende der äußeren Hülse 3 insbesondere so ausgebildet sein, dass die Ausrichteinheit 10 mit dem lichtaustrittsseitigen Ende der äußeren Hülse 3 auf das Werkstück 5 oder im Falle einer Zahnbehandlung auf den zu behandelnden Zahn oder einen Umgebungsbereich davon aufgestützt werden kann. Das lichtaustrittsseitige Ende der inneren Hülse 4 ist dann um die genannte vorgegebene Wegstrecke von dem zu behandelnden Bereich der Oberfläche des Werkstücks 5 beabstandet. Die Stirnseite der inneren Hülse 4 wird durch eine für die Laserstrahlung transparente Scheibe 7 gebildet, welche beispielsweise aus Diamantglas gefertigt sein kann.

Ein wesentlicher Vorteil der in der Fig. 1 gezeigten Anordnung besteht darin, dass der Laserstrahl 1 im Strahlengang zu der zu bearbeitenden Oberfläche innerhalb der inneren Hülse 4 geführt wird und somit vor der Umgebung geschützt ist. Es findet demnach in diesem Teil des Strahlengangs keine störende Absorption oder Streuung des Laserstrahls an Materialpartikeln, etwa an ablatierten Materialpartikeln, statt. Es ist somit anzustreben, die inneren Hülse 4 möglichst lang auszuführen, sodass der Laserstrahl 1 erst knapp oberhalb der Oberfläche des zu behandelnden Werkstücks 5 aus der inneren Hülse 4 austritt. Hierbei ist jedoch zu beachten, dass die transparente Scheibe 7 damit in den Nahbereich des Fokus des Laserstrahls 1 gerät und damit die Gefahr einer Zerstörung der transparenten Scheibe 7 aufgrund der unvermeidlich vorhandenen Restabsorption oder der Multiphotonenabsorption des Materials der transparente Scheibe 7 bei der Wellenlänge des Laserstrahls 1 besteht. Es könnte sich somit als notwendig erweisen, die transparente Scheibe 7 zumindest um die Rayleigh-Länge von der Oberfläche des zu bearbeitenden Werkstücks 5 zu beabstanden. Für die Rayleigh-Länge $z_R$ gilt:

$$z_R = \pi w_0^2 / \lambda \qquad (1)$$

wobei $w_0$ der Radius des Strahlquerschnitts im Fokus des Laserstrahls ist. Für den Radius des Strahlquerschnitts bei der Rayleigh-Länge $z_R$ gilt:

$$w(z_R) = \sqrt{2}\, w_0 \quad . \qquad (2)$$

Dies bedeutet, dass die Intensität des Laserstrahls bei der Rayleigh-Länge $z_R$ auf ca. 50% abgefallen ist.

Es kann je nach den Umständen jedoch auch möglich sein, den Abstand zwischen der transparenten Scheibe 7 und dem lichtaustrittsseitigen Ende der äußeren Hülse 3 kleiner als die Rayleigh-Länge $z_R$ zu wählen. Insbesondere wenn sich herausstellt, dass die Restabsorption oder die Multiphotonenabsorption der transparenten Scheibe 7 bei der Wellenlänge des Laserstrahls 1 unkritisch ist und zu keiner Zerstörung der transparenten Scheibe 7 führt, kann der Abstand auch kleiner als $z_R$ gewählt werden.

Ein weiterer Aspekt, der bei der Wahl des Abstands zwischen der transparenten Scheibe 7 und dem Rand der äusseren Hülse 3 eine Rolle spielt, ist die Gefahr der Verschmutzung der transparenten Scheibe 7 durch ablatiertes Material. Das ablatierte Material kann dabei insbesondere an der transparenten Scheibe 7 anhaften und somit zur Erhöhung der Absorption des Laserstrahls beitragen. Daher kann es angebracht sein, einen etwas größeren Abstand zwischen der transparenten Scheibe 7 und dem lichtaustrittsseitigen Ende der äußeren Hülse 3 zu wählen. Um der Gefahr der Verschmutzung der transparenten Scheibe 7 wirkungsvoll zu begegnen, kann jedoch auch vorgesehen sein, die transparente Scheibe 7 in situ zu reinigen, worauf weiter unten noch näher eingegangen werden wird.

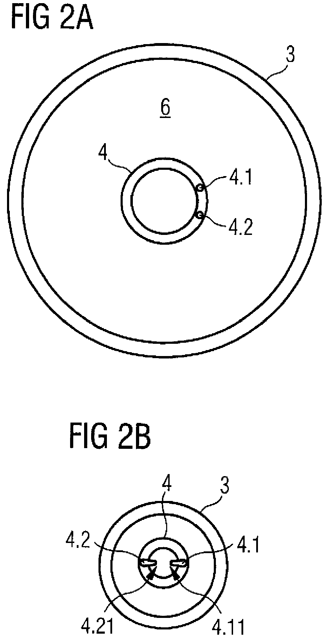

Die in der Fig. 1 dargestellte Ausführungsform einer Laserstrahl-Ausrichteinheit 10 ist ebenfalls dafür ausgelegt, über Zuleitungen, wie sie beispielhaft mit den Bezugsziffern 8 und 9 gekennzeichnet sind, weitere flüssige, gasförmige oder plasmaartige Medien dem Ort der Bearbeitung an der Oberfläche des Werkstücks 5 zuzuführen. Diese Zuleitungen 8 und 9 könnten beispielsweise durch Öffnungen in der Blende 6 geführt werden und an ihrem Ende mit geeigneten Düsen versehen sein. Diese Variante könnte jedoch mit Schwierigkeiten verbunden sein, da infolge des Vorhandenseins der inneren Hülse 4 die Medien nicht mehr unbedingt zielgerichtet mittels der Düsen auf den Ort der Bearbeitung ausgerichtet werden könnten. Die in der Fig. 1 gezeigte Ausführungsform beinhaltet daher eine Variante, bei der die Zuleitungen 8 und 9 mit Kanälen 4.1 und 4.2 verbunden sind, welche in die Wand der inneren Hülse 4 integriert sind. In den Querschnittsdarstellungen der Figuren 2A, B können die Kanäle 4.1 und 4.2 an ihrem oberen Ende, d.h. im Anfangsbereich der inneren Hülse 4 (A) und an ihrem Endpunkt, d.h. am lichtaustrittsseitigen Ende der inneren Hülse 4 (B) gesehen werden. Die Kanäle 4.1 und 4.2 können entlang dem Kegelmantel der inneren Hülse 4 als gerade verlaufende Kanäle mit im Querschnitt betrachtet konstanter Winkelposition oder als Kurve mit veränderlicher Winkelposition ausgelegt sein. An ihrem Endpunkt können die Kanäle 4.1 und 4.2 mit geeigneten steuer- und ausrichtbaren Düsen 4.11 und 4.21 verbunden sein.

Wie bereits erwähnt, können über die Zuleitungen 8 und 9 und die Kanäle 4.1 und 4.2 verschiedenartige Medien dem Bearbeitungsort auf der Oberfläche des Werkstücks 5 zugeführt werden, worauf weiter unten noch näher eingegangen werden wird. Es kann jedoch insbesondere vorgesehen sein, dass über eine Zuleitung und den mit ihr verbundenen Kanal ein Reinigungsmittel wie im einfachsten Fall Wasser zugeführt und über eine geeignete mit dem Kanal verbundene Düse über die Oberfläche der transparenten Scheibe 7 gesprüht wird, um somit die transparente Scheibe 7 von darauf abgelagerten Ablationspartikeln zu reinigen. Diese Art Reinigung kann gegebenenfalls auch in situ, d.h. während eines Ablationsvorgangs durchgeführt werden, wobei gegebenenfalls darauf geachtet werden muss, dass das Reinigungsmedium die Laserstrahlung nicht absorbiert, d.h. bei Verwendung infraroter Laserstrahlung keine nennenswerte Absorption im infraroten Spektralbereich aufweist. In der Fig. 2B ist zur Veranschaulichung gezeigt, wie mittels der Düse 4.21 ein Reinigungsmedium wie Wasser auf die äußere Oberfläche der transparenten Scheibe 7 gesprüht und die transparente Scheibe 7 somit von Ablationspartikeln gereinigt werden kann. Die Düse 4.21 kann zu diesem Zweck permanent nach innen, d.h. auf die Oberfläche der transparenten Scheibe 7 ausgerichtet sein. Die übrigen, mit entsprechenden Kanälen verbundenen Düsen können dagegen auf die zu bearbeitende Stelle auf der Oberfläche des Werkstücks 5 ausgerichtet sein.

Die äußere Hülse 3 kann, wie in dem Ausführungsbeispiel der Fig. 1 dargestellt, ebenfalls derart ausgeführt sein, dass sie eine doppelwandige Struktur aufweist, wobei die dadurch zwischen den beiden Wänden gebildete Kammer als eine Absaugleitung ausgebildet werden kann, indem sie an eine ebenfalls durch den oberen Abschnitt der Ausrichteinheit verlaufende Leitung angeschlossen wird und diese Leitung mit einer Pumpe verbunden wird. Wie in den Figuren 2A, B gezeigt, kann die Kammer 11 zylindersymmetrisch ausgebildet sein und am unteren Ende in Richtung auf den Bearbeitungsort an der Oberfläche des Werkstücks 5 ausgerichtet sein. Auf diese Weise werden ablatierte Partikel nach allen Richtungen in die Kammer 11 abgesaugt. Im oberen Bereich kann dann die Kammer 11 mit einer Leitung 12 verbunden sein.

Wie bereits erwähnt, können über die Zuführungsleitungen 8 und 9 und die Kanäle 4.1 und 4.2 verschiedenartige Substanzen wie beispielsweise organische oder anorganische Zusammensetzungen, pharmazeutische Substanzen oder im einfachsten Fall Luft oder Wasser zugeführt werden. Es können aber auch beispielsweise Photo-Sensitizer, Plasmen oder Nanopartikel als sog. Quellen freier Elektronen, sog. seed electrons, oder direkt exogen erzeugte freie Elektronen exakt dem Fokus als Ort der Bearbeitung zugeführt werden. Als pharmazeutische Substanzen können insbesondere solche Substanzen zugeführt werden, mit denen ein verbesserter Schutz des umliegenden Gewebes bei Zahnbehandlungen erreicht werden soll, wobei insbesondere das umliegende Gewebe vor bei der Gewebebearbeitung ggf. entstehendem thermischen und/oder chemischen Stress geschützt werden soll. Hierfür kommt als Substanz insbesondere Ectoin und Derivate davon in Betracht, wobei vorteilhafter Weise eine isotone Ectoin-Lösung eingesetzt werden sollte, da diese den gleichen osmotischen Druck wie menschliches Blut aufweist und somit eventuell auftretende Blutungen rasch gestillt werden könnten. Eine isotonische Ectoin-Lösung kann beispielsweise hergestellt werden, indem einer 1%-igen Ectoin-Lösung NaCl in einem Konzentrationsbereich von 0,6 - 0,7% zugefügt wird.

Gemäss einer weiteren Ausführungsform kann zwischen der äusseren Hülse 3 und der inneren Hülse 4 ein Unterdruck oder ein Vakuum erzeugt werden. Auf diese Weise kann die Lebensdauer und damit die freie Weglänge der injizierten freien exogenen Elektronen erhöht und die benötigte Lichtleistung weiter reduziert werden.

In dem in den Fig. 1 und 2A,B gezeigten Ausführungsbeispiel ist die innere Hülse 4 als ein starres und einstückig gebildetes Rohr vorgesehen. Es ist jedoch ebenso denkbar, die innere Hülse 4 nach der Art eines Teleskoprohres auszubilden, also insbesondere als ein System aus mehreren sich zylindrisch verjüngenden und parallel in sich geführten Röhren oder Zylindern, welche linear bis zu einem Anschlag auf eine maximale Auszugslänge herausgezogen und wieder ineinander zurückgeschoben werden können. Dabei kann vorgesehen sein, dass die Länge der inneren Hülse 4 automatisch veränderbar ist und insbesondere derart automatisch veränderbar ist, dass ein Abstand zwischen der Werkstückoberfläche und dem Schutzglas 7 bzw. dem unteren Ende der inneren Hülse 4 konstant gehalten wird. Dieser Abstand kann beispielsweise der bereits genannten Rayleigh-Länge entsprechen. Es kann jedoch auch jeder andere Abstand eingestellt werden, von dem bekannt ist, dass das Schutzglas jedenfalls so weit ausserhalb des Fokus liegt, dass seine Zerstörung durch den Laserstrahl ausgeschlossen ist. Die automatische Veränderung der Länge der inneren Hülse 4 kann mit der automatischen Fokussierung des Laserstrahls 50 durch die Autofokus-Einheit 2 geeignet gekoppelt sein. Jede durch die Autofokus-Einheit 2 bewirkte Veränderung der Brennweite bewirkte eine axiale Verschiebung des Laserfokus, so dass dieser wieder exakt auf der Werkstückoberfläche zu liegen kommt. Gleichermaßen wird die Länge der inneren Hülse 4 verändert, so dass der Abstand zwischen dem Schutzglas 7 und der Oberfläche bzw. dem Laserfokus gleich bleibt.

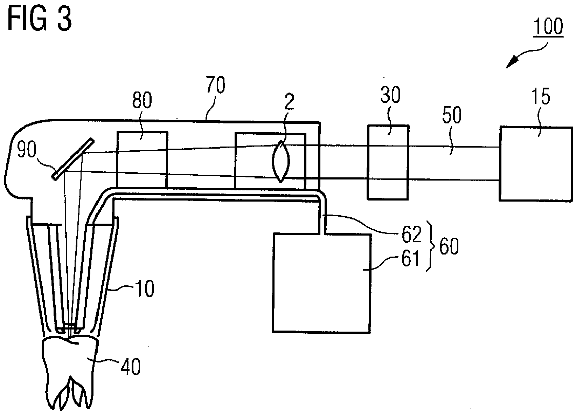

In der Fig. 3 ist eine Ausführungsform eines dentalen Laserbearbeitungsgeräts schematisch und mit nicht maßstabsgetreuen Größenverhältnissen dargestellt. Das Laserbearbeitungsgerät 100 weist eine Laserstrahlquelle 15 auf, welche einen gepulsten Bearbeitungs-Laserstrahl 50 emittiert. Der Bearbeitungs-Laserstrahl 50 wird auf die Oberfläche eines zu behandelnden Zahns 40 fokussiert. Gegebenenfalls kann der Bearbeitungs-Laserstrahl vorher durch eine Strahlformungseinheit 30 derart geformt werden, dass er ein rechteckförmiges oder Tophat-Strahlprofil erhält. Anschliessend kann der Laserstrahl 50 in ein Handstück 70 eingekoppelt werden, welches eine Autofokuseinheit 20, eine Scan-Einheit 80, eine Umlenkeinheit 90 und eine Ausrichteinheit 10 aufweist. Der Laserstrahl 50 wird innerhalb des Handstücks 70 zuerst der Autofokus-Einheit 20 mit einer Linse 2 zugeführt, durch die der Laserstrahl 50 derart auf die Zahnoberfläche fokussiert wird, dass die Oberfläche stets im Fokus des Laserstrahls 50 bleibt. Durch die Scan-Einheit 80 kann der Laserstrahl 50 über einen bestimmten Oberflächenbereich gescannt oder gerastert werden. Dann wird der Laserstrahl 50 durch eine optische Umlenkeinheit 90 wie einen Spiegel, ein Spiegelsystem aus mehreren Spiegeln oder ein Umlenkprisma in Richtung auf die Zahnoberfläche umgelenkt.

Es kann dabei vorgesehen sein, dass die Laserstrahlquelle 15 die Laserpulse solchermaßen generiert, dass diese eine Pulsdauer in einen Bereich zwischen 100 fs und 100 ns und eine Energie pro Puls in einem Bereich oberhalb 1 nJ aufweisen. Die Fokussierung des Laserstrahls 50 kann derart eingestellt werden, dass der Laserstrahl 50 auf der Oberfläche des Zahns 40 einen Fokus mit einem Fokusdurchmesser in einem Bereich von 1 μm - 100 μm aufweist. Es kann weiter vorgesehen sein, dass die Laserstrahlquelle 15 die Laserpulse mit einer Repetitionsrate in einem Bereich von 1 Hz - 10 MHz emittiert.

Das Laserbearbeitungsgerät 100 weist ferner eine Laserstrahl-Ausrichteinheit 10 auf, wie sie weiter oben im Zusammenhang mit den Figuren 1 und 2A, B beschrieben wurde und hier nicht mehr im Detail dargestellt ist.

Das Laserbearbeitungsgerät 100 weist ferner eine Ausgabeeinrichtung 60 zur Ausgabe einer Ectoin-Lösung in Richtung auf eine Umgebung des zu bearbeitenden Zahns 40 auf. Die Ausgabeeinrichtung 60 kann wie dargestellt eine Vorratskammer 61 zur Bevorratung der Ectoin-Lösung und eine mit der Vorratskammer 61 verbundene Zuleitung 62 enthalten. Die Zuleitung 62 kann dann mit einem der Kanäle der zweiten, inneren Hülse der Ausrichteinheit verbunden sein, um durch eine Düse auf eine Umgebung des zu bearbeitenden Zahns appliziert zu werden.

Es sei angemerkt, dass in dem Ausführungsbeispiel der Fig. 3 die beiden Aspekte der vorliegenden Anmeldung, nämlich der Einsatz einer Ausrichteinheit gemäß der Figuren 1 und 2A, B als auch der Einsatz einer Ausgabeeinrichtung für eine Ectoin-Lösung zusammengefasst sind. Es sei jedoch angemerkt, dass diese beiden Aspekte auch unabhängig voneinander gesehen werden können. Das Laserbearbeitungsgerät 100 kann somit eine Ausrichteinheit gemäß Figuren 1 und 2A, B aufweisen, jedoch keine Ausgabeeinrichtung für eine Ectoin-Lösung. Umgekehrt kann das Laserbearbeitungsgerät 100 eine Ausgabeeinrichtung für eine Ectoin-Lösung, jedoch keine Ausrichteinheit gemäß Fig. 1 und 2A, B aufweisen, in welch letzterem Fall das Laserbearbeitungsgerät 100 eine andere, etwa konventionelle Ausrichteinheit aufweisen kann.

Das Laserbearbeitungsgerät 100 kann des Weiteren Ausgabeeinrichtungen für andere Medien aufweisen, wie sie oben bereits beschrieben wurden. Neben konventionellen Medien wie Luft oder Wasser können dies beispielsweise Substanzen wie Photosensitizer, Plasmen oder andere Arten von Elektronenquellen sein, mit denen der Ablationsprozess unterstützt werden kann. Eine Ausgabeeinrichtung für freie Elektronen kann beispielsweise nach der Art einer Braunschen Kathodenstrahlröhre ausgebildet sein.

**Patentansprüche**

1. Laserstrahl-Ausrichteinheit zur Bearbeitung eines Materials, umfassend:

   eine äußere Hülse (3) und eine innere Hülse (4), welche innerhalb der äußeren Hülse (3) derart angeordnet ist, dass der Laserstrahl durch ihren inneren Hohlraum in Richtung auf den zu bearbeitenden Bereich des Materials führbar ist, wobei die äußere Hülse (3) das lichtaustrittsseitige Ende der inneren Hülse (4) um eine vorgegebene Wegstrecke in Achsenrichtung der Hülsen überragt,

   **dadurch gekennzeichnet, dass**

   die innere Hülse (4) an ihrem lichtaustrittsseitigen Ende mit einer für den Laserstrahl transparenten Scheibe (7) abgeschlossen ist, und die vorgegebene Wegstrecke der Rayleigh-Länge in Bezug auf den Fokus des aus dem lichtaustrittsseitigen Ende der inneren Hülse (4) austretenden Laserstrahlbündels entspricht.

2. Laserstrahl-Ausrichteinheit zur Bearbeitung eines Materials, umfassend:

   eine äußere Hülse (3) und eine innere Hülse (4), welche innerhalb der äußeren Hülse (3) derart angeordnet ist, dass der Laserstrahl durch ihren inneren Hohlraum in Richtung auf den zu bearbeitenden Bereich des Materials führbar ist,

   **dadurch gekennzeichnet, dass**

   die innere Hülse (4) an ihrem lichtaustrittsseitigen Ende mit einer für den Laserstrahl transparenten Scheibe (7) abgeschlossen ist, und die innere Hülse (4) eine Mehrzahl von Hülsen verschiedener Durchmesser aufweist, die teleskopartig derart relativ zueinander angeordnet sind, dass durch gegenseitiges Verschieben der Hülsen die Länge der inneren Hülse (4) einstellbar ist.

3. Laserstrahl-Ausrichteinheit nach Anspruch 1, **dadurch gekennzeichnet, dass** die äußere (3) und/oder die innere Hülse (4) einen sich in Richtung auf das lichtaustrittsseitige Ende stetig verringernden Durchmesser aufweist.

4. Laserstrahl-Ausrichteinheit nach einem der vorhergehenden Ansprüche, **dadurch gekennzeichnet, dass** die innere Hülse (4) an ihrem lichteintrittsseitigen Ende an einer Blende (6) befestigt ist.

**5.** Laserstrahl-Ausrichteinheit nach einem der vorhergehenden Ansprüche,
**dadurch gekennzeichnet, dass**
in eine Wand der äußeren Hülse (3) und/oder in eine Wand der inneren Hülse (4) mindestens eine Zuführungs- oder Absaugleitung integriert ist.

**6.** Laserstrahl-Ausrichteinheit nach Anspruch 5,
**dadurch gekennzeichnet, dass**
die Leitung an ihrem Ende mit einer Düse verbunden ist.

**7.** Laserstrahl-Ausrichteinheit nach einem der vorhergehenden Ansprüche,
**dadurch gekennzeichnet, dass**
die innere Hülse (4) innenseitig eine lichtreflektierende Beschichtung aufweist.

**8.** Laserstrahl-Ausrichteinheit nach einem der vorhergehenden Ansprüche,
**dadurch gekennzeichnet, dass**
die innere Hülse (4) an ihrem lichteintrittseitigen Ende offen ist.

**9.** Laserstrahl-Ausrichteinheit nach einem der vorhergehenden Ansprüche,
**dadurch gekennzeichnet, dass**
in dem inneren Hohlraum der inneren Hülse (4) ein Vakuum vorhanden oder erzeugbar ist.

**10.** Laserbearbeitungsgerät zur Bearbeitung eines Materials, umfassend:

eine Laserstrahlquelle (15) zum Bereitstellen eines gepulsten Bearbeitungs-Laserstrahls, und eine Laserstrahl-Ausrichteinheit (10) nach einem der vorherigen Ansprüche.

**11.** Laserbearbeitungsgerät nach Anspruch 10, ferner umfassend:

eine Scan-Einheit zum Abrastern oder Scannen eines Bereichs mit dem Laserstrahl, wobei die Scan-Einheit, die Führungsoptik für den Laserstrahl und die Abmessungen der inneren Hülse (4) derart aufeinander abgestimmt sind, dass der gescannte Bereich beim Durchtritt des Laserstrahls durch die innere Hülse (4) stets innerhalb der zweiten Hülse (4) zu liegen kommt.

**12.** Laserbearbeitungsgerät nach Anspruch 11,
**dadurch gekennzeichnet, dass**
das Laserbearbeitungsgerät eine Ausgabeeinrichtung (60) zur Ausgabe einer Ectoin-Lösung aufweist.

**13.** Laserbearbeitungsgerät nach einem der Ansprüche 11 oder 12, ferner umfassend:

eine Einrichtung zur Erzeugung und Zuführung freier Elektronen.

**Claims**

**1.** Laser beam aligning unit for processing a material, comprising:

an outer sleeve (3) and an inner sleeve (4), which is arranged within the outer sleeve (3) in such a way that the laser beam is guidable through its inner cavity in the direction of the region to be processed of the material, wherein the outer sleeve (3) projects beyond the light-exit-side end of the inner sleeve (4) by a predefined distance in the axial direction of the sleeves,

**characterized in that**

the inner sleeve (4) is closed off at its light-exit-side end with a plate (7) that is transparent to the laser beam, and the predefined distance corresponds to the Rayleigh length in relation to the focus of the laser beam emerging from the light-exit-side end of the inner sleeve (4).

**2.** Laser beam aligning unit for processing a material, comprising:

an outer sleeve (3) and an inner sleeve (4), which is arranged within the outer sleeve (3) in such a way that the laser beam is guidable through its inner cavity in the direction of the region to be processed of the material,

**characterized in that**

the inner sleeve (4) is closed off at its light-exit-side end with a plate (7) that is transparent to the laser beam, and the inner sleeve (4) has a plurality of sleeves of different diameters which are arranged telescopically relative to one another in such a way that the length of the inner sleeve (4) is adjustable by mutual displacement of the sleeves.

**3.** Laser beam aligning unit according to Claim 1,
**characterized in that**

the outer sleeve (3) and/or the inner sleeve (4) have/has a diameter decreasing contiguously in the direction of the light-exit-side end.

**4.** Laser beam aligning unit according to any of the preceding claims,

**characterized in that**
the inner sleeve (4) is fixed to a diaphragm (6) at its light-entrance-side end.

5. Laser beam aligning unit according to any of the preceding claims,
**characterized in that**
at least one feed or extraction line is integrated into a wall of the outer sleeve (3) and/or into a wall of the inner sleeve (4).

6. Laser beam aligning unit according to Claim 5,
**characterized in that**
the line is connected to a nozzle at its end.

7. Laser beam aligning unit according to any of the preceding claims,
**characterized in that**
the inner sleeve (4) has a light-reflecting coating on the inner side.

8. Laser beam aligning unit according to any of the preceding claims,
**characterized in that**
the inner sleeve (4) is open at its light-entrance-side end.

9. Laser beam aligning unit according to any of the preceding claims,
**characterized in that**
a vacuum is present or generatable in the inner cavity of the inner sleeve (4).

10. Laser processing apparatus for processing a material, comprising:

a laser beam source (15) for providing a pulsed processing laser beam, and
a laser beam aligning unit (10) according to any of the preceding claims.

11. Laser processing apparatus according to Claim 10, further comprising:

a scanning unit for scanning a region with the laser beam, wherein
the scanning unit, the guide optical unit for the laser beam and the dimensions of the inner sleeve (4) are coordinated with one another in such a way that, upon passage of the laser beam through the inner sleeve (4), the scanned region always becomes located within the second sleeve (4).

12. Laser processing apparatus according to Claim 11,
**characterized in that**
the laser processing apparatus has a dispensing device (60) for dispensing an ectoine solution.

13. Laser processing apparatus according to either of Claims 11 and 12, furthermore comprising:

a device for generating and feeding free electrons.

## Revendications

1. Unité d'orientation de faisceau laser pour le traitement d'une matière, comprenant :

un manchon extérieur (3) et un manchon intérieur (4) agencé de telle façon dans le manchon extérieur (3), que le faisceau laser peut être guidé à travers l'espace creux interne de celui-ci, en direction de la zone de matière à traiter, le manchon extérieur (3) faisant saillie au-delà de l'extrémité du manchon intérieur (4) côté sortie de lumière sur une distance prédéfinie dans la direction axiale des manchons,

**caractérisée en ce que**

le manchon intérieur (4) est fermé par un disque transparent au faisceau laser (7) au niveau de son extrémité côté sortie de lumière, et
la distance prédéfinie correspond à la longueur de Rayleigh par rapport au point focal du paquet de faisceaux laser sortant de l'extrémité côté sortie de lumière du manchon intérieur (4).

2. Unité d'orientation de faisceau laser pour le traitement d'une matière, comprenant:

un manchon extérieur (3) et un manchon intérieur (4) agencé de telle façon dans le manchon extérieur (3), que le faisceau laser peut être guidé à travers l'espace creux intérieur de celui-ci, en direction de la zone de matière à traiter,

**caractérisée en ce que**

le manchon intérieur (4) est fermé par un disque transparent au faisceau laser (7) au niveau de son extrémité côté sortie de lumière, et
le manchon intérieur (4) présente une pluralité de manchons de différents diamètres, agencés de façon télescopique les uns par rapport aux autres, de telle façon qu'un coulissement réciproque des manchons permet de régler la longueur du manchon intérieur (4).

3. Unité d'orientation de faisceau laser selon la revendication 1,
**caractérisée en ce que**
le manchon extérieur (3) et/ou le manchon intérieur (4) présentent un diamètre constamment décroissant en direction de l'extrémité côté sortie de lumière.

4. Unité d'orientation de faisceau laser selon l'une des revendications précédentes,
**caractérisée en ce que**
le manchon intérieur (4) est fixé à un diaphragme (6) à son extrémité côté entrée de lumière.

5. Unité d'orientation de faisceau laser selon l'une des revendications précédentes,
**caractérisée en ce qu'**
au moins une conduite d'alimentation ou d'aspiration est intégrée dans une paroi du manchon extérieur (3) et/ou dans une paroi du manchon intérieur (4).

6. Unité d'orientation de faisceau laser selon la revendication 5,
**caractérisée en ce qu'**
à son extrémité, la conduite est raccordée à une buse.

7. Unité d'orientation de faisceau laser selon l'une des revendications précédentes,
**caractérisée en ce que**
du côté intérieur, le manchon intérieur (4) présente un revêtement réfléchissant la lumière.

8. Unité d'orientation de faisceau laser selon l'une des revendications précédentes,
**caractérisée en ce que**
le manchon intérieur (4) est ouvert à son extrémité côté entrée de lumière.

9. Unité d'orientation de faisceau laser selon l'une des revendications précédentes,
**caractérisée en ce qu'**
un vide existe ou peut être créé dans l'espace creux intérieur du manchon intérieur (4).

10. Appareil de traitement laser pour le traitement d'une matière, comprenant:

une source de faisceau laser (15) destinée à fournir un faisceau laser de traitement pulsé, et une unité d'orientation de faisceau laser (10) selon l'une des revendications précédentes.

11. Appareil de traitement laser selon la revendication 10, comprenant en outre:

une unité de balayage pour l'analyse ou le balayage d'une zone avec le faisceau laser, dans lequel
l'unité de balayage, l'optique de guidage pour le faisceau laser et les dimensions du manchon intérieur (4) sont adaptées les unes aux autres de telle façon que la zone balayée s'étend toujours à l'intérieur du deuxième manchon (4) pendant le passage du faisceau laser à travers le manchon intérieur (4).

12. Appareil de traitement laser selon la revendication 11,
**caractérisée en ce que**
l'appareil de traitement laser comporte un dispositif de sortie (60) destiné à délivrer une solution d'ectoïne.

13. Appareil de traitement laser selon l'une des revendications 11 ou 12, comprenant en outre:

un dispositif destiné à produire et alimenter des électrons libres.

# FIG 1

FIG 2A

FIG 2B

FIG 3

100

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 2004176718 A1

- US RE37585 E1